# EUROPEAN PATENT APPLICATION

(11) **EP 4 524 986 A1**
(43) Date of publication of application: **19.03.2025**
(21) Application number: 23900979.8
(22) Date of filing: 27.11.2023
(51) Int. Cl.: G16H 20/70, G16H 10/60, G16H 20/30

(54) **SELF-CARE MENTAL HEALTH MANAGEMENT APPARATUS AND METHOD FOR PRESCRIBING CUSTOMIZED DIGITAL CONTENT AND INDUCING FORMATION OF BEHAVIORAL HABITS**

(30) Priority: 08.12.2022 KR 20220171030
(71) Applicant: Mamt Co., Ltd., Seongnam-si, Gyeonggi-do 13597 (KR)
(72) Inventor: HONG, Chang Hyung, Suwon-si Gyeonggi-do 16499 (KR); SON, Sang Joon, Suwon-si Gyeonggi-do 16499 (KR); ROH, Hyun Woong, Suwon-si Gyeonggi-do 16499 (KR); CHO, Yong Hyuk, Suwon-si Gyeonggi-do 16499 (KR); HONG, Sun Hwa, Suwon-si Gyeonggi-do 16499 (KR); NAM, You Jin, Suwon-si Gyeonggi-do 16499 (KR)
(74) Representative: Jung, Minkyu
(86) International application number: PCT/KR2023/019204
(87) International publication number: WO 2024/122960

(57) **Abstract**

Disclosed are a self-care mental health management apparatus and method, which prescribe customized digital content for users based on the emotion-cognition-behavior theory and induce the formation of behavioral habits for mental health management. Disclosed are a self-care mental health management apparatus and method for prescribing user-customized digital content on the basis of an emotion-recognition-behavior theory and inducing the formation of behavioral habits for mental health management. The self-care mental health management method, according to embodiments of the present invention, comprises the steps of: according to the type of mental health of a user, selecting, from among a plurality of pieces of content, emotional content, cognitive content, and behavioral content, which are to be recommended to the user by emotional category, cognitive category, and behavioral category; and determining, according to the type of mental health of the user, a recommendation order of the emotional content, cognitive content, and behavior content and providing the content to a user terminal according to the recommendation order.

## Description

### [TECHNICAL FIELD]

The present disclosure relates to a self-care mental health management apparatus and method, and more specifically, to a self-care mental health management apparatus and method that prescribes user-customized digital content based on the emotion-cognition-behavior theory and induces the formation of behavioral habits for mental health management.

The present disclosure is derived from research supported by the Ministry of Health and Welfare's R&D project to foster research-oriented hospitals (Super Gap SUPER * Senior Total Health Care Platform) and the Ministry of Science and ICT's R&D project to develop biomedical technology (Ajou SMART FARM Biocore Construction Project).

### [BACKGROUND ART]

In recent years, the number of people suffering from mental illnesses such as depression has been increasing rapidly for a variety of reasons, including an aging population, an individualized society, the rise of single-person families, social stress, and congenital and genetic factors. There are also a growing number of people who are temporarily depressed, even if they are not suffering from a mental illness. As such, there are various types of symptoms and mental illnesses related to mental health, and different types of users need customized services for mental health management. Currently, there are various applications (apps) for mental health management, but these apps are not based on medical knowledge related to mental health and simply provide content capable of providing mental comfort or causing emotional changes.

### [DETAILED DESCRIPTION OF THE INVENTION]

### [TECHINICAL PROBLEM]

Embodiments of the present disclosure provide a self-care mental health management apparatus and method, which prescribe customized digital content for users based on the emotion-cognition-behavior theory and induce the formation of behavioral habits for mental health management.

In addition, embodiments of the present disclosure provide a self-care mental health management apparatus and method, which enable users to manage their own mental health without time and space constraints through self-healthcare, and to practice and make a habit of preventing and continuously managing mental illness.

In addition, embodiments of the present disclosure provide a self-care mental health management apparatus and method, which provide customized mental health management services to various users by providing steady treatment and management to patients with mental illness to provide symptom relief and mental stability, providing screening for mental illness, in-depth checkup, comfort and sympathy, and solutions for recovery to high-risk users, and helping general users form lifestyle habits, strengthen motivation, and recognize the need for mental health management.

### [TECHNICAL SOLUTION]

According to an embodiment, a self-care mental health management method for prescribing customized digital content for mental health management of a user and inducing formation of mental health-related behavioral habits of the user includes a step (A) of accessing, by a content delivery unit, a content database in which a plurality of content provided for purpose of prescribing customized digital content and inducing formation of behavioral habits are constructed while being classified into emotional category content, cognitive category content, and behavioral category content and a step (B) of determining, by the content delivery unit, content to be recommended to the user according to a mental health profile of the user, including at least one of a mental health situation, a mental illness, and a mental health symptom of the user, and providing the content to a user terminal of the user.

The step (B) may include selecting emotional content, cognitive content, and behavioral content to be recommended to the user by emotional content category, cognitive content category, and behavioral content category among the plurality of content according to the mental health profile of the user, and determining a recommendation order of the emotional content, the cognitive content, and the behavioral content according to the mental health profile of the user and providing the content to the user terminal according to the recommendation order.

The step (B) may include selecting one or more emotional content to be recommended to the user from among a plurality of emotional category content based on the mental health profile of the user, and determining a recommendation order of the one or more emotional content, selecting one or more cognitive content to be recommended to the user from among a plurality of cognitive category content based on the mental health profile of the user, and determining a recommendation order of the one or more cognitive content, selecting one or more behavioral content to be recommended to the user from among a plurality of behavioral category content based on the mental health profile of the user, and determining a recommendation order of the one or more behavioral content, and sequentially providing the one or more emotional content, the one or more cognitive content, and the one or more behavioral content to the user terminal according to the recommendation orders.

The step (B) may include selecting one of a situation thumbnail feeding rule, an illness thumbnail feeding rule, and a symptom thumbnail feeding rule based on the mental health profile of the user, determining a chronological recommendation order of the one or more emotional content, the one or more cognitive content, and the one or more behavioral content based on the mental health profile of the user according to the thumbnail feeding rule, and sequentially providing the one or more emotional content, the one or more cognitive content, and the one or more behavioral content to the user terminal according to the chronological recommendation order.

The emotional category content may be content corresponding to at least one sub-emotional content category among comfort, interest stimulation, empathy, and encouragement related to the user's mental health among the plurality of content, the cognitive category content may be content corresponding to at least one sub-cognitive content category among advice, motivation, consolation, and illness education related to the user's mental health among the plurality of content, and the behavioral category content may be content corresponding to at least one sub-behavioral content category among indoor/outdoor activities, hobby recommendation, self-observation, and volunteering related to the user's mental health among the plurality of content.

At least one of the emotional content category, the cognitive content category and the behavioral content category may be classified into a plurality of category levels based on a plurality of sub-emotion categories, a plurality of sub-cognition categories, or a plurality of sub-behavior categories which are subcategories of the category.

The step (B) may include determining a recommendation order of content corresponding to the at least one category according to the mental health profile of the user based on the plurality of category levels, and the recommendation order of the plurality of category levels may be determined differently according to the situation thumbnail feeding rule, the illness thumbnail feeding rule, and the symptom thumbnail feeding rule.

The step (B) may include classifying the mental health profile of the user based on the user's gender, age, environment, situation, personality, amount of exercise/activity, type of stress management, mental illness history, and mental health-related symptom.

The self-care mental health management method may further include providing, by the content delivery unit, a mission for mental health management to the user, and providing a raffle ticket for the user to participate in a raffle for mental health hero characters when the user completes the mission using the user terminal, providing one or more mental health hero characters of a plurality of predetermined mental health hero characters to the user terminal if the user wins the raffle based on the raffle ticket, and providing a reward to the user when the user has collected all mental health hero characters included in a preset collection of mental health hero characters.

According to an embodiment, there is provided a non-transitory computer-readable storage medium having recorded thereon a computer program for executing the self-care mental health management method.

According to an embodiment, a self-care mental health management apparatus for prescribing customized digital content for mental health management of a user and inducing formation of mental health-related behavioral habits of the user includes a content database constructed such that a plurality of content provided for purpose of prescribing customized digital content and inducing formation of behavioral habits are classified into emotional category content, cognitive category content, and behavioral category content, and a content delivery unit that determines content to be recommended to the user according to a mental health profile of the user, including at least one of a mental health situation, a mental illness, and a mental health symptom of the user, and provides the content to a user terminal of the user..

The content delivery unit may select emotional content, cognitive content, and behavioral content to be recommended to the user by emotional content category, cognitive content category, and behavioral content category among the plurality of content according to the mental health profile of the user, and determine a recommendation order of the emotional content, the cognitive content and the behavioral content according to the mental health profile of the user and provide the content to the user terminal according to the recommendation order.

The content delivery unit may select one or more emotional content to be recommended to the user from among a plurality of emotional category content based on the mental health profile of the user, and determine a recommendation order of the one or more emotional content, select one or more cognitive content to be recommended to the user from among a plurality of cognitive category content based on the mental health profile of the user, and determine a recommendation order of the one or more cognitive content, select one or more behavioral content to be recommended to the user from among a plurality of behavioral category content based on the mental health profile of the user, and determine a recommendation order of the one or more behavioral content, and sequentially provide the one or more emotional content, the one or more cognitive content, and the one or more behavioral content to the user terminal according to the recommendation orders.

The content delivery unit may select one of a situation thumbnail feeding rule, an illness thumbnail feeding rule, and a symptom thumbnail feeding rule based on the mental health profile of the user, determine a chronological recommendation order of the one or more emotional content, the one or more cognitive content, and the one or more behavioral content based on the mental health profile of the user according to the thumbnail feeding rule, and sequentially provide the one or more emotional content, the one or more cognitive content, and the one or more behavioral content to the user terminal according to the chronological recommendation order.

The content delivery unit may determine a recommendation order of content corresponding to the at least one category according to the mental health profile of the user based on the plurality of category levels. The recommendation order of the plurality of category levels may be determined differently according to the situation thumbnail feeding rule, the illness thumbnail feeding rule, and the symptom thumbnail feeding rule.

The content delivery unit may analyze the mental health profile of the user based on the user's gender, age, environment, situation, personality, amount of exercise/activity, type of stress management, mental illness history, and mental health-related symptom.

The content delivery unit may provide a mission for mental health management to the user, and providing a raffle ticket for the user to participate in a raffle for mental health hero characters when the user completes the mission using the user terminal, provide one or more mental health hero characters of a plurality of predetermined mental health hero characters to the user terminal if the user wins the raffle based on the raffle ticket, and provide a reward to the user when the user has collected all mental health hero characters included in a preset collection of mental health hero characters.

### [ADVANTAGEOUS EFFECTS OF THE INVENTION]

According to the embodiments of the present disclosure, it is possible to provide a self-care mental health management apparatus and method, which prescribe customized digital content for users based on the emotion-cognition-behavior theory and induce the formation of behavioral habits for mental health management.

Further, according to the embodiments of the present disclosure, it is possible to provide a self-care mental health management apparatus and method, which enable users to manage their own mental health without time and space constraints through self-healthcare, and to practice and make a habit of preventing and continuously managing mental illness.

Further, according to the embodiments of the present disclosure, it is possible to provide a self-care mental health management apparatus and method, which provide customized mental health management services to various users by providing steady treatment and management to patients with mental illness to provide symptom relief and mental stability, providing screening for mental illness, in-depth checkup, comfort and sympathy, and solutions for recovery to high-risk users, and helping general users form lifestyle habits, strengthen motivation, and recognize the need for mental health management.

### [DESCRIPTION OF THE DRAWINGS]

FIG. 1 is a configuration diagram showing a self-care mental health management system including a self-care mental health management apparatus according to an embodiment of the present disclosure.
FIG. 2 is a flow chart of a self-care mental health management method according to an embodiment of the present disclosure.
FIG. 3 is a configuration diagram of a content delivery unit constituting a self-care mental health management apparatus according to an embodiment of the present disclosure.
FIG. 4 is a flowchart showing step S120 of FIG. 2.
FIG. 5 is an example diagram of a situation thumbnail feeding rule determined according to a self-care mental health management method according to an embodiment of the present disclosure.
FIG. 6 is an example diagram of an illness thumbnail feeding rule determined according to a self-care mental health management method according to an embodiment of the present disclosure.
FIG. 7 is a flowchart for describing functions of a reward management unit constituting a self-care mental health management apparatus according to an embodiment of the present disclosure. of the present disclosure.
FIG. 8 is an example diagram showing an execution screen of a self-care mental health management app displayed on a user terminal according to an embodiment of the present disclosure.
FIG. 9 is an example diagram showing a user interface screen of a mental state recording unit among the execution screens of the self-care mental health management app shown in FIG. 8.
FIG. 10 is an example diagram showing the classification items of situational video content provided in a self-care mental health management app according to an embodiment of the present disclosure.
FIG. 11 is an example diagram showing the classification items of symptom-specific video content provided in a self-care mental health management app according to an embodiment of the present disclosure.
FIG. 12 is an example diagram showing a detail screen of video prescription provided by the content delivery unit provided by a self-care mental health management app according to an embodiment of the present disclosure.
FIGS. 13 and 14 are example diagrams showing the execution screen of a behavioral habit induction unit provided by a self-care mental health management app according to an embodiment of the present disclosure.
FIGS. 15 and 16 are example diagrams showing an execution screen of a customized prescription unit provided by a self-care mental health management app according to an embodiment of the present disclosure.
FIG. 17 is an example diagram showing an execution screen of a comprehensive checkup unit provided by a self-care mental health management app according to an embodiment of the present disclosure.
FIG. 18 is an example diagram showing a mental health management calendar and content storage provided by a self-care mental health management app according to an embodiment of the present disclosure.
FIG. 19 is a diagram showing an example of an auxiliary menu item provided by a self-care mental health management app according to an embodiment of the present disclosure.
FIGS. 20 and 21 are diagrams illustrating an execution screen of a mission performance reward provision unit provided by a self-care mental health management app according to an embodiment of the present disclosure.

### [BEST MODE]

Advantages and features of the present disclosure and methods for achieving them will be apparent with reference to embodiments described below in detail in conjunction with the accompanying raffles. However, the present disclosure is not limited to the embodiments disclosed below, but may be implemented in various forms, and these embodiments are to make the disclosure of the present disclosure complete, and are provided so that this disclosure will be thorough and complete and will fully convey the scope of the invention to those of ordinary skill in the art, which is to be defined only by the scope of the claims. Like reference numerals refer to like elements throughout the specification.

A self-care mental health management apparatus and method for prescribing customized digital content and inducing the formation of mental health-related behavioral habits of a user according to an embodiment of the present disclosure may determine content to be recommended to a user according to a user mental health profile including at least one of the user's mental health condition, mental illness, and mental health symptom based on the emotion-cognition-behavior theory, and provides the content to a user terminal.

A self-care mental health management apparatus and method according to an embodiment of the present disclosure may select one or more emotional content, one or more cognitive content, and one or more behavioral content to be recommended to a user for each of an emotional content category, a cognitive content category, and a behavioral content category among a plurality of content according to a user's mental health profile, determine a recommendation order of the one or more emotional content, the one or more cognitive content, and the one or more behavioral content according to the user's mental health profile, and provide content to a user terminal according to the recommendation order.

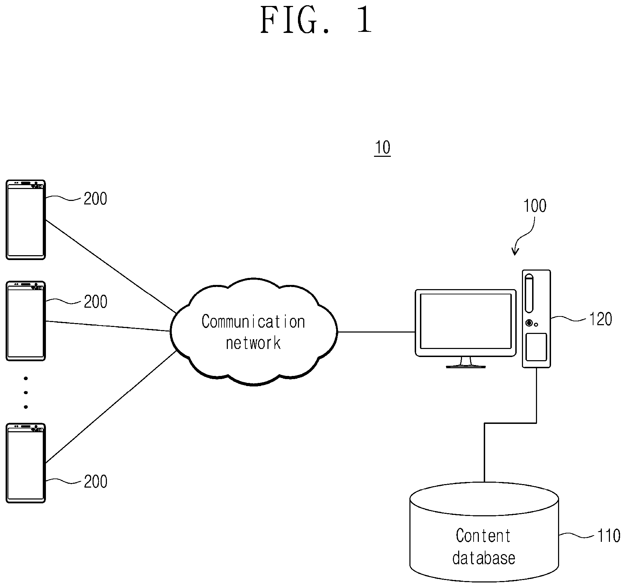

FIG. 1 is a configuration diagram showing a self-care mental health management system including a self-care mental health management apparatus according to an embodiment of the present disclosure. Referring to FIG. 1, a self-care mental health management system 10 according to an embodiment of the present disclosure may include a self-care mental health management apparatus 100. The self-care mental health management apparatus 100 may be provided as a self-care mental health management server that provides a service for prescribing customized digital content for the mental health management of a user and inducing the formation of behavioral habits related to the user's mental health to a user terminal 200.

The self-care mental health management apparatus 100 according to an embodiment of the present disclosure may include a content database 110 in which a plurality of content for prescribing customized digital content and inducing the formation of behavioral habits are stored, and a content delivery unit 120 that provides customized content for prescribing mental health and inducing the formation of behavioral habits according to the user's mental health profile among the plurality of content stored in the content database 110.

In order to provide customized content according to the emotion-cognition-behavior theory, the content database 110 may be constructed such that a plurality of content is classified into emotional category content, cognitive category content, and behavioral category content. The content delivery unit 120 may determine content to be recommended to the user according to the user's mental health profile including the user's mental health condition, mental illness, and/or mental health symptom, and provide the content to the user's user terminal 200. The user terminal 200 may be a terminal used by a user who receives a mental health management service, and may be provided as a mobile terminal such as a smartphone or smart pad, a desktop PC, a notebook, a tablet PC, a laptop PC, or the like but is not limited thereto.

The content database 110 may store a variety of content such as videos pre-produced by a manager for mental health management, YouTube videos for new health management, social network (SNS) content such as Instagram-toon (for example, illustrations or webtoons directly uploaded by individual creators through SNS channels such as Instagram), movies, digital books, content applications, games, and the like, by emotion, cognition, and behavior categories.

The emotional category content may be content corresponding to at least one sub-emotional content category among comfort, interest stimulation, empathy, and encouragement related to the user's mental health among a plurality of content. The cognitive category content may be content corresponding to at least one sub-cognitive content category among advice, motivation, consolation, and illness education related to the user's mental health among a plurality of content. The behavioral category content may be content corresponding to at least one sub-behavioral content category among indoor/outdoor activities, hobby recommendation, self-observation, and volunteering related to the user's mental health among a plurality of content.

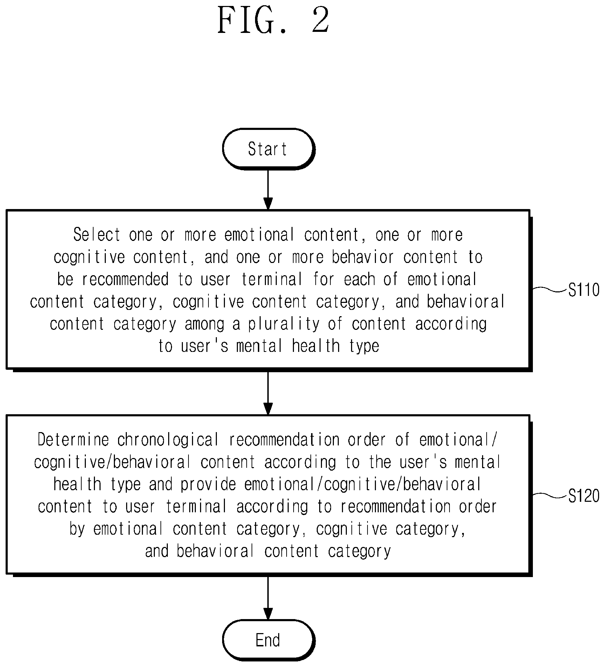

FIG. 2 is a flow chart of a self-care mental health management method according to an embodiment of the present disclosure. Referring to FIG. 1 and FIG. 2, a content delivery unit 120 may access the content database 110 in which a plurality of content provided for prescribing customized digital content and inducing the formation of behavioral habits are classified into emotional category content, cognitive category content, and behavioral category content, select one or more emotional content, one or more cognitive content, and one or more behavioral content to be recommended to the user for each of the emotional content category, the cognitive content category, and the behavioral content category among a plurality of content according to the user's mental health profile including the user's mental health condition, mental illness, and/or mental health symptom, determine a chronological recommendation order of the selected one or more emotional content, the selected one or more cognitive content, and the selected one or more behavioral content, and provide the content to the user terminal 200 according to the recommendation order.

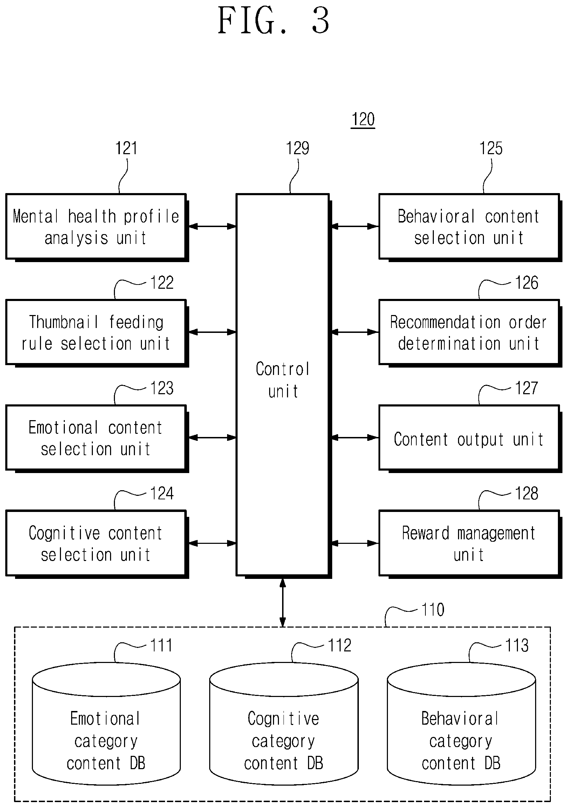

FIG. 3 is a configuration diagram of a content delivery unit constituting the self-care mental health management apparatus according to an embodiment of the present disclosure. Referring to FIGS. 1 to 3, the content delivery unit 120 may include a mental health profile analysis unit 121, a thumbnail feeding rule selection unit 122, an emotional content selection unit 123, a cognitive content selection unit 124, a behavioral content selection unit 125, a recommendation order determination unit 126, a content output unit 127, a reward management unit 128, and a control unit 129.

The mental health profile analysis unit 121 may be configured to analyze the user's mental health profile based on user personal characteristic information including the user's gender (female, male), age (teens, 20s, 30s, 40s, 50s, 60s or 60+), environment, situation, personality (introverted, extroverted), amount of exercise/activity, stress management type/tendency (courageous, cheerful, serious, diligent), mental illness history, and/or mental health-related symptoms by a mental health profile analysis artificial intelligence model. The user personal characteristic information may be obtained by user input through the user terminal 200, may be obtained through various sensors (acceleration sensor, gyro sensor, etc.) provided to the user terminal 200, or may be provided from a medical institution terminal (medical server) through a communication network.

User medical information input by the user into a health management app on the user terminal or stored in the medical institution terminal (medical institution server) may be included in or added to the user personal characteristic information, and the user personal characteristic information may include, for example, resilience-related information (such as thoughts about happiness or finding meaning in life, courage, self-efficacy, etc.), personality traits (such as stress, low self-esteem, negative thinking, excessive thinking and rumination, interpersonal relationships, self-deprecation/self-hatred, self-doubt/disappointment, etc.), general symptoms (such as anxiety, insomnia, lethargy, nightmares, oversleeping, helplessness, disengagement, emptiness, frustration, burnout, emotionlessness, fear, obsession, avoidance of others, harsh speech, confusion, difficulty concentrating, mood swings, overwhelm, psychological pressure, mourning, isolation, irritability, tension, nervousness, fear, anger, avoidance, impulsive behavior, excessive pushing, excessive guilt, self-blame, regret, loss, somatization), severe symptoms (such as delusions, hallucinations, cognitive distortions, ego-dissociation, delirium, loss of appetite, binge eating, anorexia, compulsions, alcohol withdrawal symptoms, forgetfulness, memory issues, etc.), and information related to mental illnesses (such as depression, depressive disorder, schizophrenia, bipolar disorder, antisocial personality disorder, borderline personality disorder, anger control disorder, anxiety disorder, panic disorder, ADHD, autism spectrum disorder, dementia, mild cognitive impairment, substance addictions such as alcohol/nicotine, gambling addiction, anger syndrome, various other addictions, PTSD, trauma, disaster situations, bereavement, suicidal thoughts, suicide attempts, self-harm, etc.).

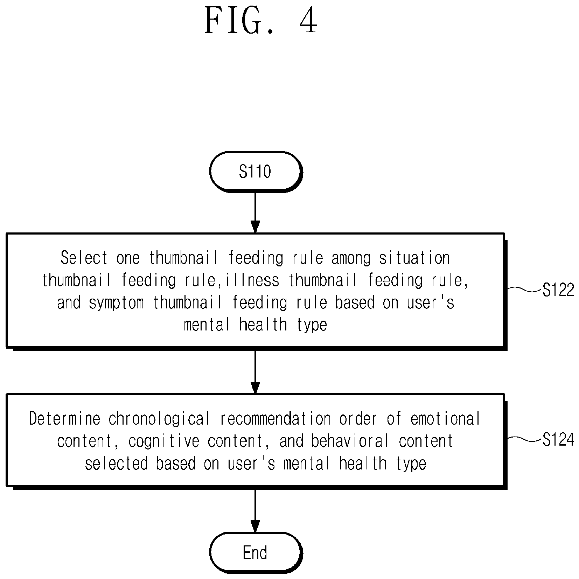

FIG. 4 is a flowchart showing step S120 of FIG. 2. Referring to FIGS. 1 to 4, in order to provide optimal customized digital content to a user and induce the user to develop appropriate behavioral habits, the thumbnail feeding rule selection unit 122 may select a thumbnail feeding rule that defines a rule for providing content to the user by a thumbnail feeding rule selection AI model (S122). In an embodiment, the thumbnail feeding rule selection unit 122 may select one of thumbnail feeding rules among situation thumbnail feeding rules, illness thumbnail feeding rules, and symptom thumbnail feeding rules based on the user's mental health profile.

The thumbnail feeding rule selection unit 122 may select a thumbnail feeding rule based on the unique characteristic information of the user, and for example, if it is determined that mental health management for the user is required due to a situation the user has experienced in the past or is currently experiencing, select the situation thumbnail feeding rule from among the situation thumbnail feeding rules, the illness thumbnail feeding rules, and the symptom thumbnail feeding rules.

As another example, if it is determined that the user is suffering from a mental health-related illness based on the user-specific personal characteristic information, the thumbnail feeding rule selection unit 122 may select the illness thumbnail feeding rule from among the situation thumbnail feeding rules, the illness thumbnail feeding rules, and the symptom thumbnail feeding rules, and if it is determined that the user has a mental health-related symptom, select the symptom thumbnail feeding rule from among the situation thumbnail feeding rules, the illness thumbnail feeding rules, and the symptom thumbnail feeding rules.

The content database 110 may include an emotional category content DB 111 that stores a plurality of emotional category content, a cognitive category content DB 112 that stores a plurality of cognitive category content, and a behavioral category content DB 113 that stores a plurality of behavioral category content. The content may be classified and stored into at least one category among the emotion-cognition-behavior categories, and one content may be stored while being repeatedly classified into two or more categories.

The emotion-cognition-behavioral content category classification of content may be automatically performed by a category classification artificial intelligence model based on the type of object in a video included in the content, as well as the text or sentences displayed in the video, voice/audio data, and so on. The category classification artificial intelligence model may be trained by supervised learning based on labeled learning data. The category classification artificial intelligence model may be implemented with a convolutional neural network (CNN), a fully connected neural network, or the like. Alternatively, the category classification of content may be performed manually by a manager.

The emotional content selection unit 123 may select one or more emotional content to recommend to the user from among a plurality of emotional category content based on the user's mental health profile. The cognitive content selection unit 124 may select one or more cognitive content to recommend to the user from among a plurality of cognitive category content based on the user's mental health profile. The behavioral content selection unit 125 may select one or more cognitive content to recommend to the user from among a plurality of behavioral category content based on the user's mental health profile.

The emotional content selection unit 123, the cognitive content selection unit 124, and the behavioral content selection unit 125 may be implemented based on the content selection artificial intelligence model. The content selection artificial intelligence model may be trained by supervised learning based on learning data. The content selection artificial intelligence model may be implemented with a convolutional neural network that analyzes information of content to select content suitable for the user, a deep neural network in which input layers and output layers are connected in a fully connected layer structure via a plurality of hidden layers and/or the like.

To the input layers, content and/or tag information of the content, unique characteristic information of the user, mental health profile analyzed by the mental health profile analysis AI model based on the information, thumbnail feeding rules analyzed by the thumbnail feeding rule selection AI model. The output layers of the content selection AI model may include the selection results of emotional content, cognitive content, and behavioral content among a plurality of content.

The recommendation order determination unit 126 may determine the recommendation order of the emotional content, cognitive content, and behavioral content, selected by the emotional content selection unit 123, cognitive content selection unit 124, and behavioral content selection unit 125 respectively, via a content recommendation order determination AI model (S 124). The recommendation order determination unit 126 may determine the chronological recommendation order of one or more emotional content, one or more cognitive content, and one or more behavioral content based on the mental health profile of the user according to the thumbnail feeding rule selected by the thumbnail feeding rule selection unit 122.

The recommendation order determination unit 126 may sequentially provide one or more emotional content, one or more cognitive content, and one or more behavioral content selected from a plurality of content based on the user's unique personal characteristic information to the user terminal 200 via the content output unit 127 according to the chronological recommendation order determined by the recommendation order determination unit 126. The user may receive the emotional content, cognitive content, and behavioral content recommended by each the emotional content category, the cognitive content category, and the behavioral content category through the user terminal 200.

At least one category among the emotional content category, the cognitive content category, and the behavioral content category may be classified into a plurality of category levels based on its subcategories: a plurality of sub-emotion categories (e.g., comfort, interest stimulation, empathy, or encouragement), a plurality of sub-cognition categories (e.g., advice related to mental health, motivation, consolation, or illness education), or a plurality of sub-behavior categories (e.g., indoor/outdoor activities related to mental health, hobby recommendation, self-observation, or volunteering).

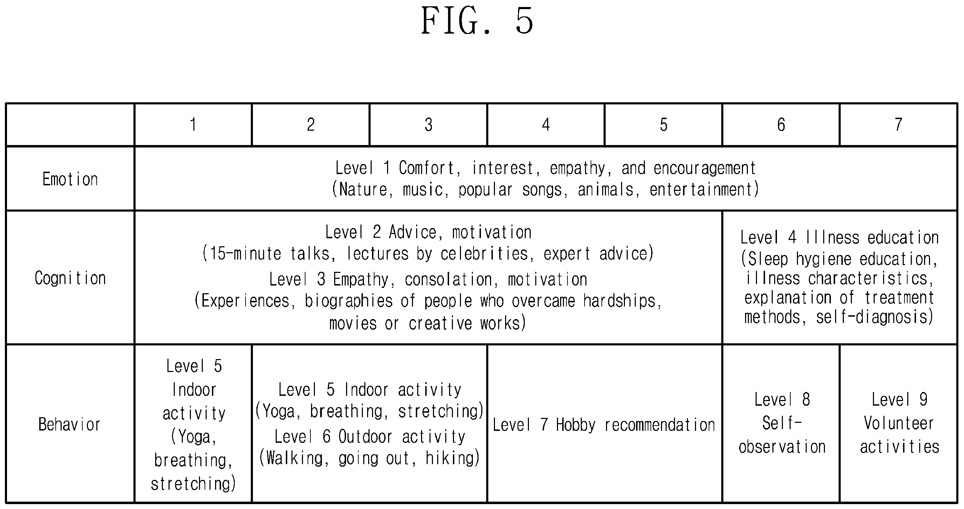

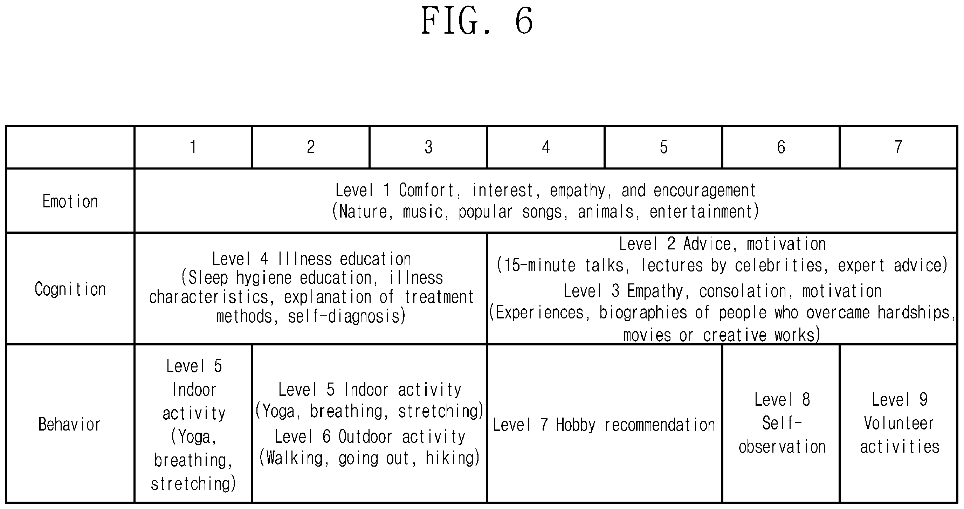

FIG. 5 is an example diagram of a situation thumbnail feeding rule determined according to a self-care mental health management method according to an embodiment of the present disclosure. FIG. 6 is an example diagram of an illness thumbnail feeding rule determined according to a self-care mental health management method according to an embodiment of the present disclosure. Referring to FIGS. 1, 2, 5, and 6, the recommendation order determination unit 126 may determine the recommendation order of content corresponding to at least one category of the emotional content category, the cognitive content category, and the behavioral content category, according to a plurality of category levels according to the user's mental health profile.

The thumbnail feeding rule may have a horizontal axis representing time (daily, weekly, monthly, and other time units), and recommended content may be provided to the user terminal in a category-level unit according to the emotional content category, the cognitive content category, and the behavioral content category, in chronological order. In the examples shown in FIGS. 5 and 6, the content is classified into a total of nine category levels. However, the number and classification of category levels are provided as examples and may vary in different implementations. In the examples shown in FIGS. 5 and 6, the first category level (Level 1) of the emotional content category may include sub-emotion categories related to comfort, sensory stimulation, emotional response induction, interest arousal, empathy, consolation, opening the heart, encouragement, and may be content such as nature, music, popular songs, flowers, animals, children, comedy, and entertainment.

Among the three category levels corresponding to the cognitive content category, the second category level (Level 2) may be content such as accurate information delivery, advice, motivation, happiness documentaries corresponding to freedom from preconceptions and misunderstandings, lectures by celebrities, advice from experts or celebrities, motivational lectures. The third category level (Level 3) may be content such as experiences corresponding to empathy, consolation, or motivation, success stories, cases or biographies of people who overcame hardships or great people, people who overcame depression, movies or creative works that overcame hardships. The fourth category level (Level 4) may be content related to illness education or drug/counseling treatment, such as drug education, providing information on depression, and providing methods of receiving counseling, or direct and practical medical education and explanations about the illness (including cognitive behavioral therapy), for example, sleep hygiene education, illness characteristics, explanation of treatment methods, self-diagnosis, or the like.

Among the five category levels corresponding to the activity category, the fifth category level (Level 5) may be content such as yoga, breathing, and stretching corresponding to indoor activities, the sixth category level (Level 6) may be content such as mountaineering, going out, and hiking corresponding to outdoor activities, the seventh category level (Level 7) may be content related to hobby recommendations, the eighth category level (Level 8) may be content related to self-observation, and the ninth category level (Level 9) may be content related to volunteering.

For example, the fifth category level (Level 5) may be content related to easy breathing methods, meditation, yoga, stretching, muscle relaxation, or home training for easy-to-following steps for moving or exercising muscles at home. The sixth category level (Level 6) may be content related to the benefits of a walk around the neighborhood, hidden attractions in the residential area, and sports for following steps for outdoor activities (moving outside, activities of going out into the outdoor environment, and making excuses to go out).

The seventh category level (Level 7) may be content related to various hobbies for me (hobbies that users are able to try themselves), such as origami, calligraphy, watercolor painting, games, quizzes, photography, cooking, and camping. The eighth category level (Level 8) may be content such as video diary writing, gratitude diary, change diary writing, and schedule stamps for self-objectification/self-observation/self-declaration (self-reflection, self-objectification, observation). The ninth category level (Level 9) may be content such as volunteer activities, gift giving, complimenting, and talent donation for activities for others, social recognition (social activities that users can try themselves or hobbies that they give to others).

The recommendation order of a plurality of category levels corresponding to at least one of the emotional content category, the cognitive content category, and the behavioral content category may be determined differently according to the situation thumbnail feeding rule, the illness thumbnail feeding rule, and the symptom thumbnail feeding rule. **In** the examples of FIGS. 5 and 6, the recommendation order of multiple cognitive content corresponding to the cognitive content category among the emotional content category, the cognitive content category, and the behavioral content category may be determined differently.

Meanwhile, a situation thumbnail with high relevance to the user's age group may be selected and content may be provided, and a thumbnail, symptom code, or the like is entered for each content, so that the content may be provided to a user through the app according to the priority order whenever the user presses a refresh button. For example, in the case of the cognitive content category, content in which the symptom code of an illness related to the user is entered may be provided first, and then all motivational videos available may be provided. **In** the case of the behavioral content category, content that matches the user's stress management type may be provided first, and then all available content may be provided. **In** the case of hobby recommendation activity and volunteer activity recommendation level, matching may be made based on the user's stress management type tag and content may be then provided sequentially according to priority.

For example, for users in their teens, thumbnail numbers may be assigned according to sub-categories such as academics, conflicts (family relationships, friends/acquaintances, alienation), addictions (media addiction, other mental health issues, or the like), and recommended content may be provided according to the emotion-cognition-behavioral content category. For users in their 20s and 30s, thumbnail numbers may be assigned according to sub-categories such as employment/job search, breakup/alienation, conflicts (family relationships, boss-colleague relationship issues), postpartum depression, and parenting stress, and emotion-cognition-behavior content may be provided accordingly.

Thumbnail numbers may be individually assigned according to sub-categories, such as conflicts with a spouse or children, addictions (alcohol, cigarettes, drinking, or the like) for users in their 40s, according to economic issues (economic hardship, debt), role changes (anxiety about retirement), family relationships/alienation, or the like for users in their 50s, and according to family relationship issues, child issues, physical health issues, bereavement, or the like for users in their 60s or older, and accordingly, content may be provided to the user terminal in a chronological recommendation order by emotion-cognition-behavioral content category.

Regardless of age group, thumbnail numbers may be assigned for common categories such as infectious diseases (COVID-19, etc.), trauma, suicidal thoughts, and suicide attempts, and emotion-cognition-behavior content may be recommended accordingly, and for illness-related categories, thumbnail numbers may be separately assigned for depression, anxiety disorder, schizophrenia, sleep disorders, bipolar disorder, dementia, ADHD, eating disorders (binge eating, anorexia), and bipolar disorder, and emotion-cognition-behavior content may be recommended accordingly. Meanwhile, more than two thumbnail numbers may be assigned to each user, and the priority (recommendation order) of the content may be determined based on the need for health management, severity of symptoms/illness, or the like, and content may be provided according to the recommendation order.

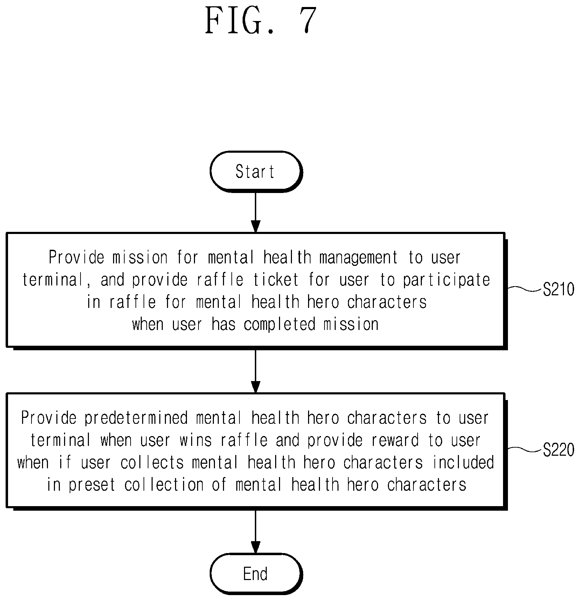

FIG. 7 is a flowchart for describing functions of a reward management unit constituting a self-care mental health management apparatus according to an embodiment of the present disclosure. of the present disclosure. Referring to FIGS. 1, 3, and 7, the reward management unit 128 may provide a mission for mental health management to a user, and provide a raffle ticket for the user to participate in a raffle for mental health hero characters when the user has completed the mission using the user terminal 200 (S210).

In addition, the reward management unit 128 may provide one or more mental health hero characters of a plurality of predetermined mental health hero characters to the user terminal 200 if the user wins the raffle based on the raffle ticket. If the user collects all mental health hero characters included in a preset collection of mental health hero characters, a reward may be provided to the user (S220).

The control unit 129 may include at least one processor that executes a mental health management program to provide a mental health management service. The control unit 129 may control the mental health profile analysis unit 121, the thumbnail feeding rule selection unit 122, the emotional content selection unit 123, the cognitive content selection unit 124, a behavioral content selection unit 125, the recommendation order determination unit 126, the content output unit 127, the reward management unit 128 to execute various functions for mental health management.

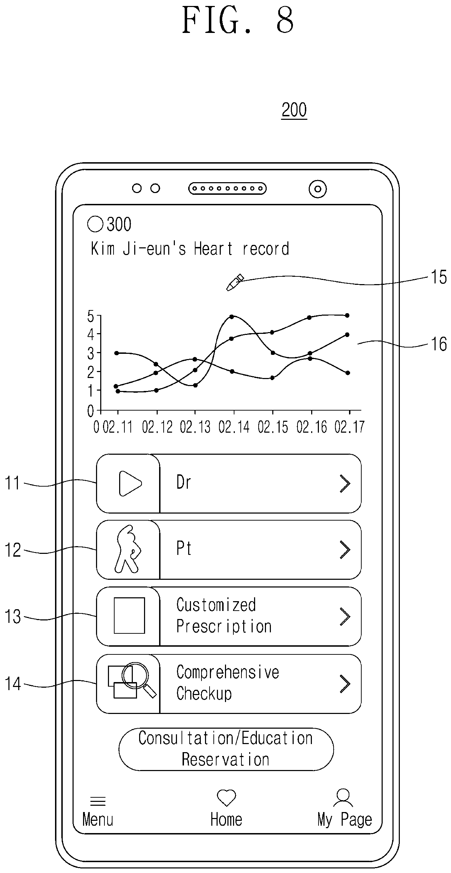

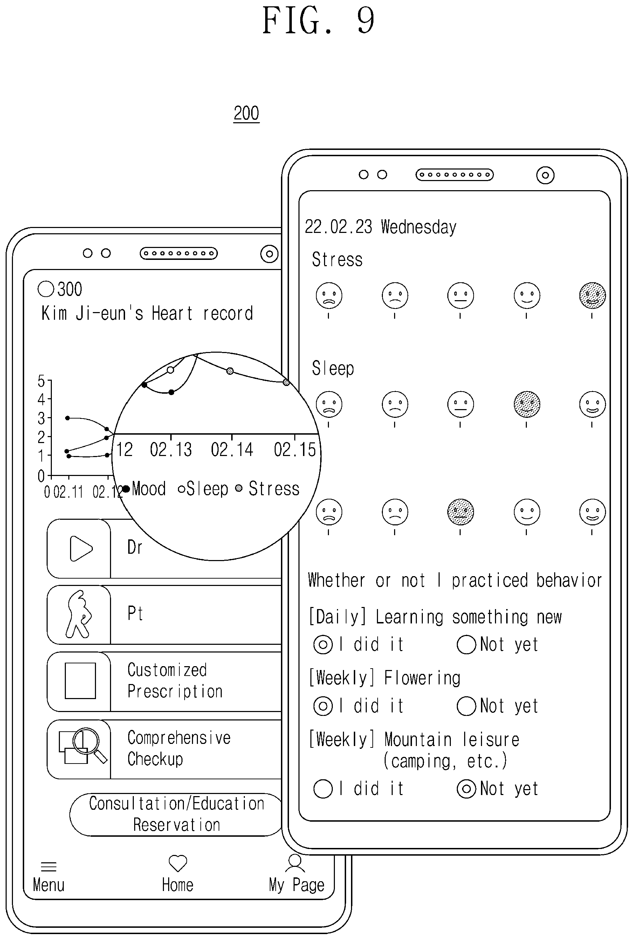

Hereinafter, a self-care mental health management apparatus and a self-care mental health management method according to an embodiment of the present disclosure will be described in detail using a self-care mental health management app as an example. FIG. 8 is an example diagram showing an execution screen of a self-care mental health management app displayed on a user terminal according to an embodiment of the present disclosure. FIG. 9 is an example diagram showing a user interface screen of a mental state recording unit among the execution screens of the self-care mental health management app shown in FIG. 8.

Referring to FIGS. 8 and 9, the execution screen of the self-care mental health management app according to the embodiment of the present disclosure may provide a mental health diagnosis section 11, a behavioral habit induction section 12, a customized prescription section 13, a comprehensive checkup section 14, a mental state record section 15, and a mental state display section 16. The mental health diagnosis section 11 and the customized prescription section 13 may provide the user terminal 200 with non-harmful content planned, reviewed, and classified based on thousands of mental illness counseling cases, provide customized content including situational/symptom-specific solution videos composed by mental health experts based on the brain science/medical theory of the emotion-cognition-behavior theory, and recommend and manage user activities as well as content such as videos focusing on practice such as empathy, advice provision, and stress management methods.

The behavioral habit induction section 12 may provide behavioral habit formation coaching recommended by experts for each practice period of daily/weekly/monthly in order to induce the formation of user's behavioral habits, provide mind control training such as a compliment diary/gratitude diary through a digital healthcare app that enhances the benefit of self-care to provide mental health training that increases the ability to respond to and build resilience against mental illness, and manage the records thereof.

The comprehensive checkup section 14 may perform a quick checkup capable of determining a user's mental health state within a short period of time of a few minutes and a verified in-depth checkup, provide an expert management service near the user's residence based on checkup results, and provide an active communication service such as partnerships with relevant institutions to ensure that user in other regions are informed of necessary mental health services.

The mental state record section 15 may enable a user to record the three key factors of biorhythm (mood, stress, and sleep) daily, allowing the user to recognize changes in their mental state. The user's mental state recorded by the mental state record section 15 may be used as an evaluation index through the user's recent records during counseling and treatment. In addition to the state evaluation, it is possible to provide a highly effective digital care service through behavioral evaluation such as a compliment diary/gratitude diary, habit practice, and viewing of video solutions.

In order to improve the user's motivation and sense of accomplishment, rewards for various items may be provided through automatic authentication when the user makes an activity, and the items subject to rewards may include a quick checkup, compliment diary, gratitude diary, behavioral practice, attendance, stress-sleep-mood evaluation, and viewing of emotional/cognitive/behavioral content (video). The mental state evaluation index recorded by the mental state record section 15 may be displayed in chronological order through the mental state display section 16.

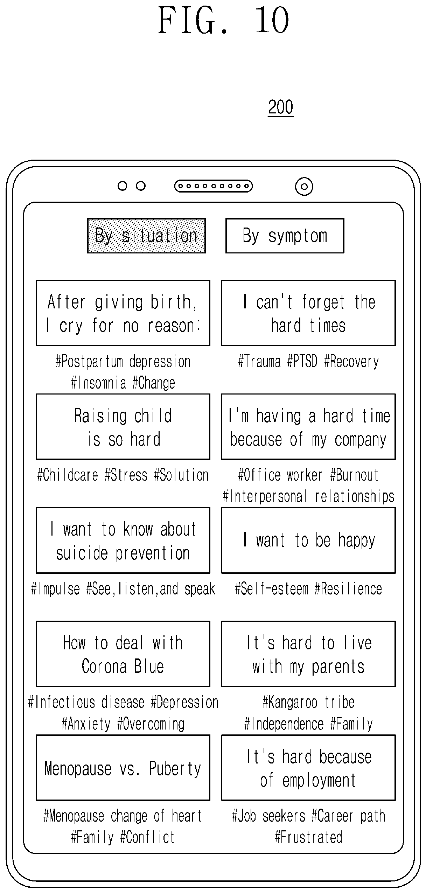

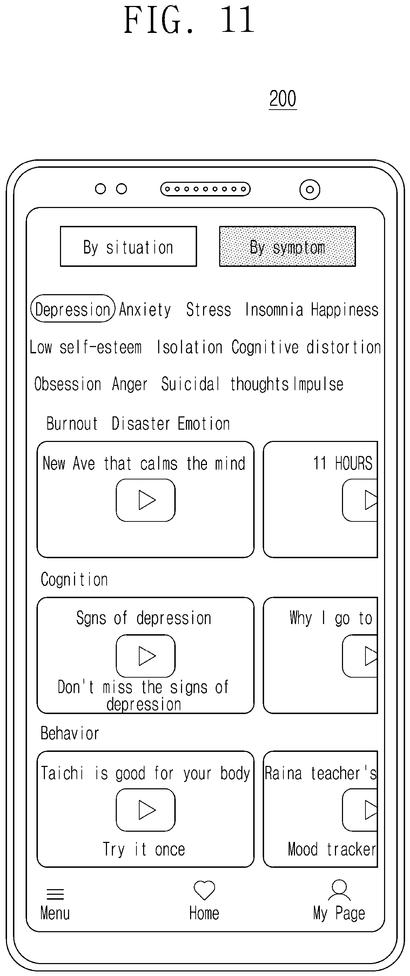

FIG. 10 is an example diagram showing the classification items of situational video content provided in a self-care mental health management app according to an embodiment of the present disclosure. FIG. 11 is an example diagram showing the classification items of symptom-specific video content provided in the self-care mental health management app according to an embodiment of the present disclosure. The self-care mental health management app according to an embodiment of the present disclosure displays the top counseling cases by situation/symptom in order of the number of counseling cases in the age group corresponding to the user, and when the user selects a counseling case of interest, video content related to the selected counseling case may be recommended. When the user inputs basic information such as gender or age and symptoms/illnesses related to mental illness, the content delivery unit (video content recommendation unit for each situation and/or video content recommendation unit for each symptom) of the self-care mental health management app may provide customized content to the user in priority order.

Major symptoms related to symptom-specific video content may include depression, anxiety, stress, insomnia, unhappiness, low self-esteem, isolation, cognitive distortion, obsession, anger, suicidal thoughts, addiction, burnout, and disaster situations. The content delivery unit may allow keywords that match the user's situation or symptoms to be displayed first at the top and recommend video content related to prevention and disease management, treatment/management methods for various mental health and illness symptoms such as depression, anxiety, stress, and insomnia. The content delivery unit may provide supplementary treatment benefits such as symptom relief by enabling a user to restore psychological stability from anxiety or depression and learning coping methods through a three-stage digital solution of emotion-cognition-behavior.

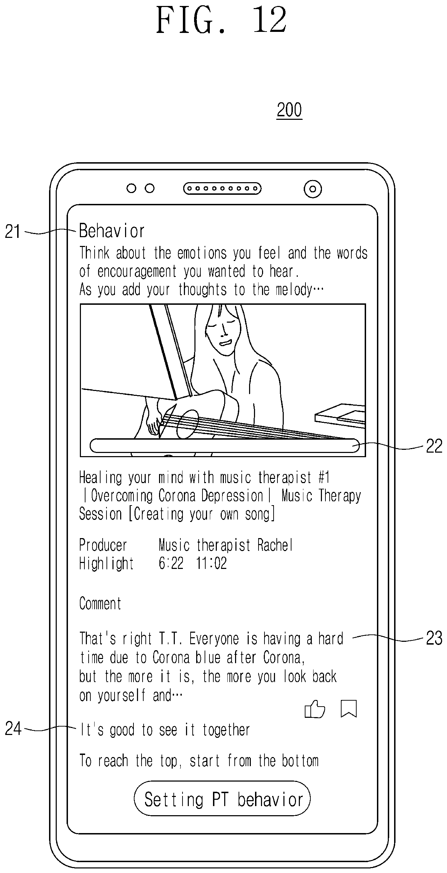

FIG. 12 is an example diagram showing a detail screen of video prescription provided by the content delivery unit provided by a self-care mental health management app according to an embodiment of the present disclosure. A classification notation item 21 indicating one of the categories (classifications) of emotion/cognition/behavior is displayed on the screen of the user terminal 200, and the video title, creator, highlight section (playback section), YouTube video comments, or the like may be additionally displayed on the classification notation item 21.

When a thumbnail item 22 is clicked on the user terminal 200, video content corresponding to the corresponding emotion, cognition, or behavioral content category may be played back. In addition, the user may click the star rating/bookmark icon 23 after watching the video to register a star rating or store the corresponding solution video in the My Page-Content Storage. In addition, a quote provision unit 24 may be additionally provided to display quotes for promoting happiness/positive encouragement along with the video content on the screen of the user terminal 200.

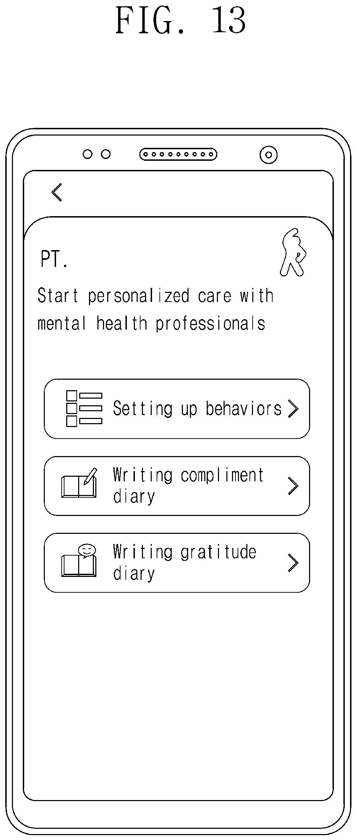

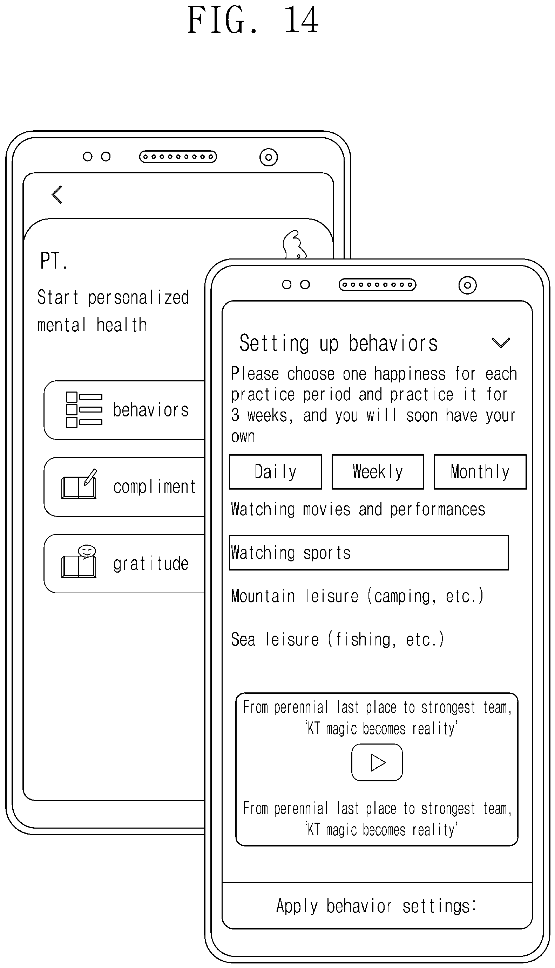

FIGS. 13 and 14 are example diagrams showing the execution screen of a behavioral habit induction unit provided by the self-care mental health management app according to an embodiment of the present disclosure. In order to manage the user's stress and induce practice, the behavioral habit induction unit may provide behavioral habit coaching recommended by experts for practice periods such as daily/weekly/monthly. Daily behavioral habit coaching items may include, for example, exercise (home training, weight training), taking a walk, taking care of a pet, running, growing plants, listening to music, recording emotions, learning something new, stretching, dancing, sleep training, practicing yoga, light physical activity, meditating, and eating fruits and vegetables. The behavioral habit induction unit may guide a user through a guide video on how to write a diary. For example, users may read past compliment diaries/gratitude diaries to reflect on how they deal with, feel, and think in various situations in their lives, and consistently practice emotional control training.

Weekly behavioral habit coaching items may include include activities such as calligraphy, cycling, coloring, checking in with others by phone, cooking, writing, taking pictures, playing musical instruments (e.g., kalimba), swimming (recreational), raffle, puzzle board games, recycling crafts, flower arranging, knitting, and transcription. Monthly behavioral habit coaching items may include volunteer activities, traveling alone, experiencing culture (such as art museums), reading a book, hiking, donating talent, traveling together, cleaning and organizing, eating out with family, going to see scenery (such as sunsets), writing letters, watching movies, watching performances, watching sports, mountain leisure (such as camping), and ocean leisure (such as fishing).

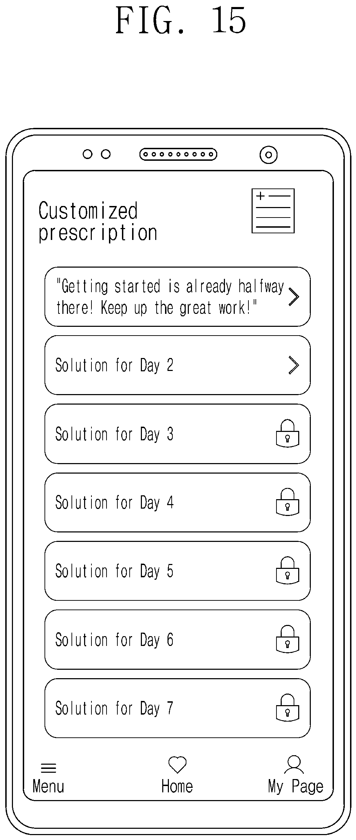

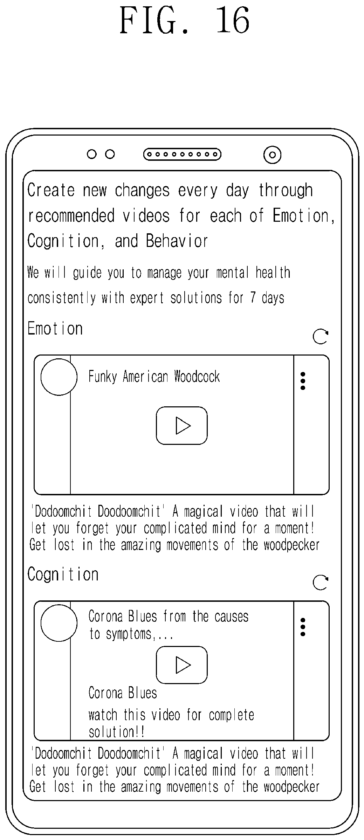

FIGS. 15 and 16 are example diagrams showing an execution screen of a customized prescription unit provided by a self-care mental health management app according to an embodiment of the present disclosure. **In** a symptom-specific solution, keywords that match a user's situation or symptoms are first exposed at the top based on diagnosis results, and when the user selects a customized solution he or she needs, a digital solution having three stages of emotion-cognition-behavior may be carried out for a set period (for example, one week) to allow the user to regain psychological stability from anxiety or depression and learn coping methods. Through this, it is possible to provide auxiliary treatment benefits such as alleviation of symptoms such as anxiety and depression.

**In** this case, the content delivery unit may perform recommendation/prescription of digital content based on the emotion-cognition-behavior theory, and chronologically provide the user terminal with a combination of 'emotion' content for the user's stability and ventilation, 'cognition' content to educate the user about the problem situation and mental illness symptoms, and 'behavior' content to enable the user to follow the stress management method to form behavioral habits according to the recommendation order.

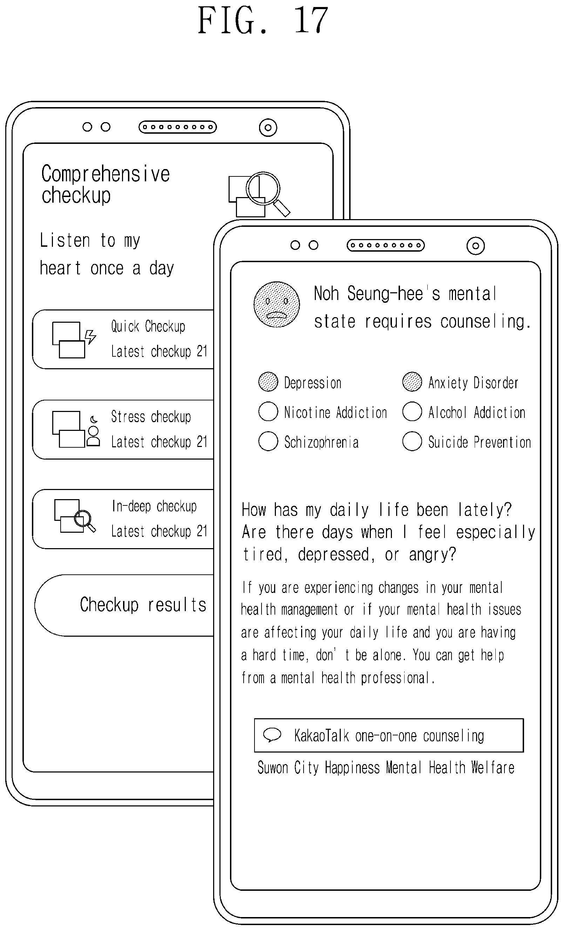

FIG. 17 is an example diagram showing an execution screen of a comprehensive checkup unit provided by the self-care mental health management app according to an embodiment of the present disclosure. The comprehensive checkup unit may include a quick checkup unit, a stress checkup unit, and an in-depth checkup unit. The quick checkup unit may diagnose major mental illnesses by life cycle and may be provided to allow checkups to be conducted within 3 minutes for children, adults, and the elderly. The stress checkup unit may identify the user's type-cheerful, brave, diligent, or serious-through a stress type test (e.g., S-LAB (Stress Level and Behavior)), recommend a suitable stress management method, and provide guides to allow users to easily practice the stress management method in their daily lives. The in-depth checkup unit may perform in-depth checkups based on the depression assessment tool (PHQ-9), the Korean version of the Personal Stress Scale (PPS), and the Generalized Anxiety Disorder-7(GAD-7). The in-depth checkup unit may provide a function of automatically notifying an checkup schedule when the user makes a counseling reservation, and allow pre- and post-checkups to be conducted continuously based on an in-depth health questionnaire.

Meanwhile, a counseling education reservation service may be used by anyone who is an app user by applying a membership system for continuous management (simplifying the process by logging in to SNS), and may be used to conduct online or offline expert counseling or participate in mental health promotion programs and mental illness prevention education by being affiliated with a mental health welfare center service. A user may check a reservation time through construction of an online system and reserve counseling education, and obtain the user's mental health evaluation index based on the results of the self-checkup conducted through the app for use in on-site counseling education.

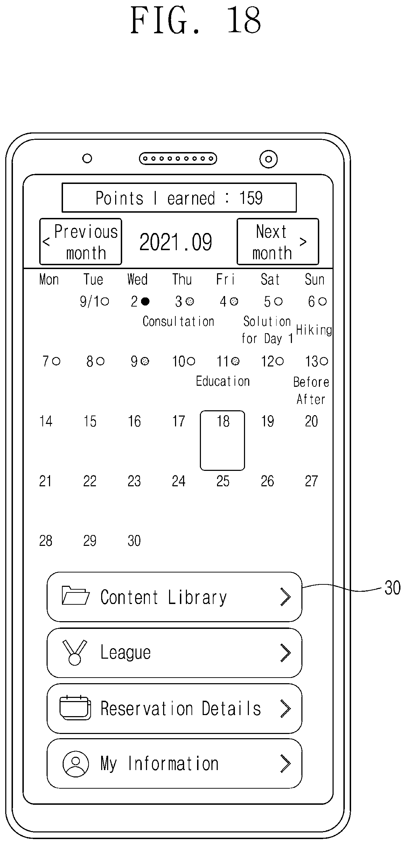

FIG. 18 is an example diagram showing a mental health management calendar and content storage provided by a self-care mental health management app according to an embodiment of the present disclosure. The user's condition, as determined by the health checkup, is displayed on the mental health management calendar with a traffic light indicator (red, yellow, green, etc.), and behavioral habits to be practiced, expert solutions or counseling/training appointments for a set period of time, or pre/post checkup schedules, may be provided through the mental health management calendar.

Active users may be rewarded with points to encourage them to continue accumulating mental assets. When a user saves a video by pressing a bookmark icon, the user may view the video in the My Page-Content Library 30. The My Page - Content Library 30 may be accessed through a top tab that categorizes emotional, cognitive, and behavioral solutions. The user may also view the satisfaction rating (star rating) of the saved video solution after watching the video, and share the video with friends on social media through a share function.

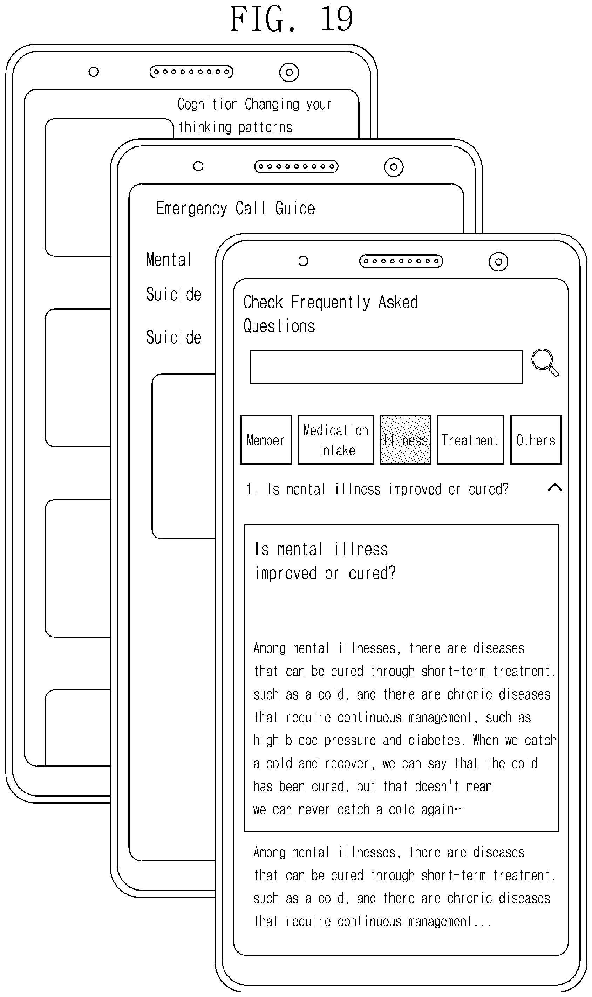

FIG. 19 is a diagram showing an example of an auxiliary menu item provided by a self-care mental health management app according to an embodiment of the present disclosure. The auxiliary menu item may allow a user to identify information about all notifications at a glance and automatically dial the emergency number of an emergency agency when clicking on the emergency call item. Also, through the auxiliary menu item, the user may view the contents of mental health information and services such as one-on-one real-time confidential counseling via <KakaoTalk Counseling>, <FAQ>, <Center Introduction>, <Announcements>, reading materials for mental health promotion like <Happiness Letter>, <App User Guide>, and mental health network resources like <Relevant Organization Guide>."

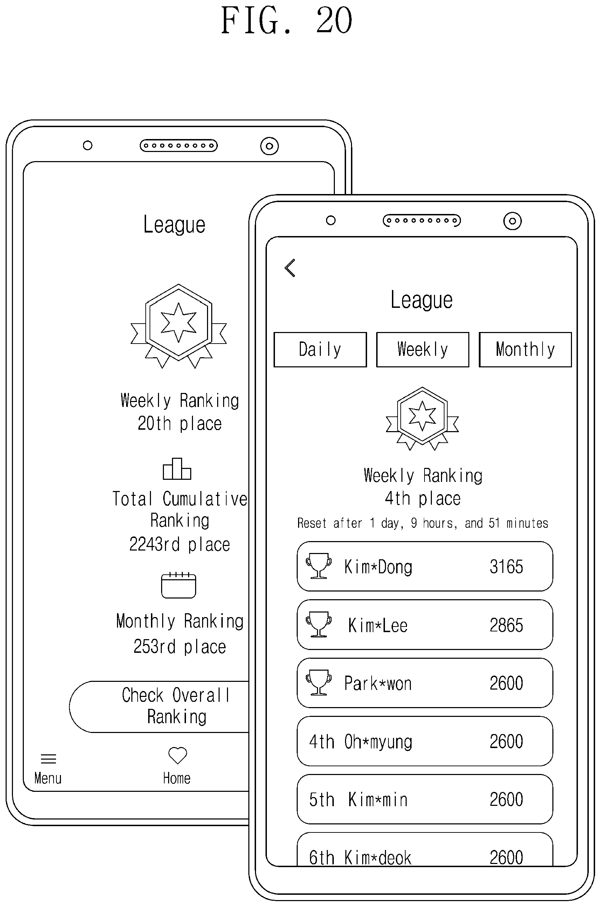

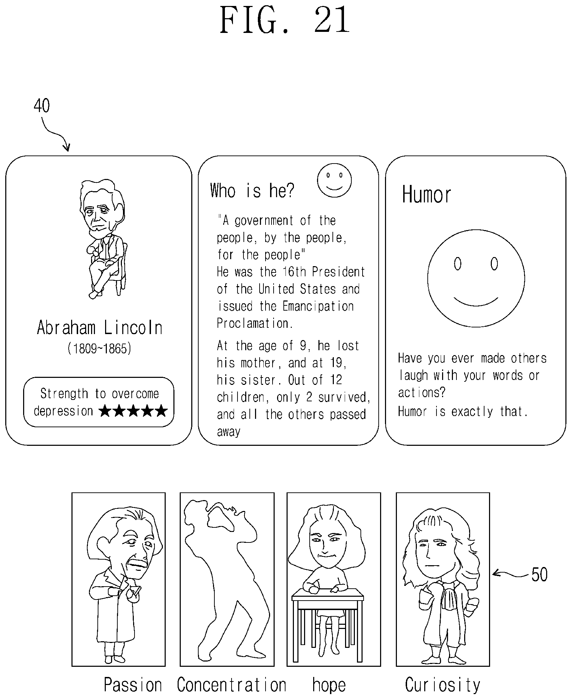

FIGS. 20 and 21 are diagrams illustrating an execution screen of a mission accomplishment reward provision unit provided by a self-care mental health management app according to an embodiment of the present disclosure. The mission accomplishment reward provision unit may help a user recognize the importance of maintaining a healthy mind to increase her or his ability to respond to and build resilience against mental illness as an asset, and provide points/rewards to encourage and motivate the user to stay active.

By operating weekly, monthly, and overall rankings and providing rewards to a user when the user has been ranked for a certain period of time, it is possible to encourage the user to use the app more actively. Rewards may be provided for attendance, quick checkup, watching videos on emotion, cognition, and behavior, compliment diary, gratitude diary, behavioral practice, stress, sleep, mood, and watching 7-day solution videos.

The mission accomplishment reward provision unit may utilize gamification principles to motivate users. The reward management unit may provide a mission game to a user terminal, and when a user completes the mission game, provide the user with a raffle ticket to be entered into a raffle for mental health hero characters. The user may utilize the raffle ticket to enter the raffle for mental health hero characters, and if the user wins the raffle, a mental health hero character 40 or 50 (e.g., a hero or great person such as Lincoln, Einstein, or Newton) may be provided to the user terminal. When the user has completed collecting all the mental health hero characters corresponding to the collection of mental health hero characters, the user may be rewarded with merchandise or the like.

According to the self-care mental health management apparatus and method according to the embodiment of the present disclosure, it is possible to provide a personalized solution for mental health management with high accuracy in various categories through machine learning based on user information. The self-care mental health management apparatus and method according to the embodiment of the present disclosure may provide a more effective self-care service to a user by first exposing customized content according to the user's preference, situation, illness, symptom, or the like, and reflecting a genre selected by the user (documentary and news, education, lecture, experience, counseling, creative works such as movies, self-care tutorials, music, explanation, game, nature, audiobook, entertainment, self-diagnosis, vlog, etc.).

**In** addition, according to the embodiment of the present disclosure, it is possible to manage high-risk groups and illness groups based on the checkup results of all users, and help with mental health management and mental illness prevention not only for users in the high-risk group or illness group but also for users in the general group. **In** addition, repetitive training and management may be facilitated through a customized solution by providing appropriate video solutions or checkup notifications via an optimized automation program, thus helping users manage their mental health. Additionally, it is possible to allow users to accumulate points through various mental health management activities, and induce the users to make an effort and engage in practice (provide motivation) through a league for all users (ranking system).

As used throughout this specification, '~ unit' is a unit that processes at least one function or operation, and may refer to, for example, a software component, FPGA, or a hardware component. The functions provided by the '~unit' may be performed separately by multiple components, or may be integrated with other additional components. The '~unit' of this specification is not necessarily limited to software or hardware, and may be configured to be on an addressable storage medium, or may be configured to play one or more processors.

The embodiments described herein may be implemented with hardware components and software components and/or a combination of the hardware components and the software components. For example, the apparatus, method and components described in the embodiments may be implemented using one or more general-purpose or special purpose computers, such as a processor, a controller and an arithmetic logic unit (ALU), a digital signal processor, a microcomputer, a field programmable array (FPGA), a programmable logic unit (PLU), a microprocessor or any other device capable of executing and responding to instructions.

The processing device may run an operating system (OS) and one or more software applications that run on the OS. The processing device also may access, store, manipulate, process, and create data in response to execution of the software. For convenience of understanding, one processing device is described as being used, but those skilled in the art will appreciate that the processing device includes a plurality of processing elements and/or multiple types of processing elements.

For example, the processing device may include multiple processors or a single processor and a single controller. In addition, different processing configurations are possible, such as parallel processors. The software may include a computer program, a piece of code, an instruction, or some combination thereof, for independently or collectively instructing or configuring the processing device to operate as desired.

Software and/or data may be embodied in any type of machine, component, physical or virtual equipment, computer storage medium or device that is capable of providing instructions or data to or being interpreted by the processing device. The software also may be distributed over network coupled computer systems so that the software is stored and executed in a distributed fashion. In particular, the software and data may be stored by one or more computer readable storage mediums.

The above-described methods may be embodied in the form of program instructions that can be executed by various computer means and recorded on a computer-readable medium. The computer readable medium may include program instructions, data files, data structures, and the like, alone or in combination. Program instructions recorded on the media may be those specially designed and constructed for the purposes of the inventive concept, or they may be of the kind well-known and available to those having skill in the computer software arts.

Examples of computer readable recording media include magnetic media such as hard disks, floppy disks and magnetic tape, optical media such as CD-ROMs, DVDs, and hardware devices specifically configured to store and execute program instructions, such as ROM, RAM, flash memory, and the like. Examples of program instructions include not only machine code generated by a compiler, but also high-level language code that can be executed by a computer using an interpreter or the like. The hardware device described above may be configured to operate as one or more software modules to perform the operations of the present disclosure, and vice versa.

Although the embodiments have been described by the limited embodiments and the drawings as described above, various modifications and variations are possible to those skilled in the art from the above description. For example, the described techniques may be performed in a different order than the described method, and/or components of the described systems, structures, devices, circuits, etc. may be combined or combined in a different form than the described method, or other components, or even when replaced or substituted by equivalents, an appropriate result can be achieved. Therefore, other implementations, other embodiments, and equivalents to the claims are within the scope of the following claims.

### [DESCRIPTION OF SYMBOLS]

10: self-care mental health management system
100: self-care mental health management apparatus
110: content database
111: emotional category content DB
112: cognitive category content DB
113: behavioral category content DB
120: content delivery unit
121: mental health profile analysis unit
122: thumbnail feeding rule selection unit
123: emotional content selection unit
124: cognitive content selection unit
125: behavioral content selection unit
126: recommendation order determination unit
127: content output unit
128: reward management unit
129: control unit
200: user terminal

## Claims

1. A self-care mental health management method for prescribing customized digital content for mental health management of a user and inducing formation of mental health-related behavioral habits of the user,
a step (A) of accessing, by a content delivery unit, a content database in which a plurality of content provided for purpose of prescribing customized digital content and inducing formation of behavioral habits are constructed while being classified into emotional category content, cognitive category content, and behavioral category content; and
a step (B) of determining, by the content delivery unit, content to be recommended to the user according to a mental health profile of the user, including at least one of a mental health situation, a mental illness, and a mental health symptom of the user, and providing the content to a user terminal of the user,
wherein the step (B) includes,
selecting emotional content, cognitive content, and behavioral content to be recommended to the user by emotional content category, cognitive content category, and behavioral content category among the plurality of content according to the mental health profile of the user; and
determining a recommendation order of the emotional content, the cognitive content, and the behavioral content according to the mental health profile of the user and providing the content to the user terminal according to the recommendation order.

2. The self-care mental health management method of claim 1, wherein the step (B) includes:
selecting one or more emotional content to be recommended to the user from among a plurality of emotional category content based on the mental health profile of the user, and determining a recommendation order of the one or more emotional content;
selecting one or more cognitive content to be recommended to the user from among a plurality of cognitive category content based on the mental health profile of the user, and determining a recommendation order of the one or more cognitive content;
selecting one or more behavioral content to be recommended to the user from among a plurality of behavioral category content based on the mental health profile of the user, and determining a recommendation order of the one or more behavioral content; and
sequentially providing the one or more emotional content, the one or more cognitive content, and the one or more behavioral content to the user terminal according to the recommendation orders.

3. The self-care mental health management method of claim 2, wherein the step (B) includes:
selecting one of a situation thumbnail feeding rule, an illness thumbnail feeding rule, and a symptom thumbnail feeding rule based on the mental health profile of the user;
determining a chronological recommendation order of the one or more emotional content, the one or more cognitive content, and the one or more behavioral content based on the mental health profile of the user according to the thumbnail feeding rule; and
sequentially providing the one or more emotional content, the one or more cognitive content, and the one or more behavioral content to the user terminal according to the chronological recommendation order.

4. The self-care mental health management method of claim 3, wherein the emotional category content is content corresponding to at least one sub-emotional content category among comfort, interest stimulation, empathy, and encouragement related to the user's mental health among the plurality of content,
wherein the cognitive category content is content corresponding to at least one sub-cognitive content category among advice, motivation, consolation, and illness education related to the user's mental health among the plurality of content, and
wherein the behavioral category content is content corresponding to at least one sub-behavioral content category among indoor/outdoor activities, hobby recommendation, self-observation, and volunteering related to the user's mental health among the plurality of content.

5. The self-care mental health management method of claim 4, wherein at least one of the emotional content category, the cognitive content category, and the behavioral content category is classified into a plurality of category levels based on a plurality of sub-emotion categories, a plurality of sub-cognition categories, or a plurality of sub-behavior categories which are subcategories of the category.

6. The self-care mental health management method of claim 5, wherein the step (B) includes determining a recommendation order of content corresponding to the at least one category according to the mental health profile of the user based on the plurality of category levels, and
wherein the recommendation order of the plurality of category levels is determined differently according to the situation thumbnail feeding rule, the illness thumbnail feeding rule, and the symptom thumbnail feeding rule.

7. The self-care mental health management method of claim 1, wherein the step (B) includes classifying the mental health profile of the user based on the user's gender, age, environment, situation, personality, amount of exercise/activity, type of stress management, mental illness history, and mental health-related symptom.

8. The self-care mental health management method of claim 1, further comprising:
providing, by the content delivery unit, a mission for mental health management to the user, and providing a raffle ticket for the user to participate in a raffle for mental health hero characters when the user completes the mission using the user terminal;
providing one or more mental health hero characters of a plurality of predetermined mental health hero characters to the user terminal if the user wins the raffle based on the raffle ticket; and
providing a reward to the user when the user has collected all mental health hero characters included in a preset collection of mental health hero characters.

9. A non-transitory computer-readable storage medium having recorded thereon a computer program for executing the self-care mental health management method of claim 1.

10. A self-care mental health management apparatus for prescribing customized digital content for mental health management of a user and inducing formation of mental health-related behavioral habits of the user,
a content database constructed such that a plurality of content provided for purpose of prescribing customized digital content and inducing formation of behavioral habits are classified into emotional category content, cognitive category content, and behavioral category content; and
a content delivery unit configured to determine content to be recommended to the user according to a mental health profile of the user, including at least one of a mental health situation, a mental illness, and a mental health symptom of the user, and provide the content to a user terminal of the user,
wherein the content delivery unit is configured to:
select emotional content, cognitive content, and behavioral content to be recommended to the user by emotional content category, cognitive content category, and behavioral content category among the plurality of content according to the mental health profile of the user; and
determine a recommendation order of the emotional content, the cognitive content, and the behavioral content according to the mental health profile of the user and provide the content to the user terminal according to the recommendation order.

11. The self-care mental health management apparatus of claim 10, wherein the content delivery unit is configured to:
select one or more emotional content to be recommended to the user from among a plurality of emotional content category content based on the mental health profile of the user, and determine a recommendation order of the one or more emotional content;
select one or more cognitive content to be recommended to the user from among a plurality of cognitive content category content based on the mental health profile of the user, and determine a recommendation order of the one or more cognitive content;
select one or more behavioral content to be recommended to the user from among a plurality of behavioral category content based on the mental health profile of the user, and determine a recommendation order of the one or more behavioral content; and
sequentially provide the one or more emotional content, the one or more cognitive content, and the one or more behavioral content to the user terminal according to the recommendation orders.

12. The self-care mental health management apparatus of claim 11, wherein the content delivery unit is configured to:
select one of a situation thumbnail feeding rule, an illness thumbnail feeding rule, and a symptom thumbnail feeding rule based on the mental health profile of the user;
determine a chronological recommendation order of the one or more emotional content, the one or more cognitive content, and the one or more behavioral content based on the mental health profile of the user according to the thumbnail feeding rule; and
sequentially provide the one or more emotional content, the one or more cognitive content, and the one or more behavioral content to the user terminal according to the chronological recommendation order.

13. The self-care mental health management apparatus of claim 12, wherein the emotional category content is content corresponding to at least one sub-emotional content category among comfort, interest stimulation, empathy, and encouragement related to the user's mental health among the plurality of content,
wherein the cognitive category content is content corresponding to at least one sub-cognitive content category among advice, motivation, consolation, and illness education related to the user's mental health among the plurality of content, and
wherein the behavioral category content is content corresponding to at least one sub-behavioral content category among indoor/outdoor activities, hobby recommendation, self-observation, and volunteering related to the user's mental health among the plurality of content.

14. The self-care mental health management apparatus of claim 13, wherein at least one of the emotional content category, the cognitive content category, and the behavioral content category is classified into a plurality of category levels based on a plurality of sub-emotion categories, a plurality of sub-cognition categories, or a plurality of sub-behavior categories which are subcategories of the category.

15. The self-care mental health management apparatus of claim 14, wherein the content delivery unit is configured to determine a recommendation order of content corresponding to the at least one category according to the mental health profile of the user based on the plurality of category levels, and
wherein the recommendation order of the plurality of category levels is determined differently according to the situation thumbnail feeding rule, the illness thumbnail feeding rule, and the symptom thumbnail feeding rule.

16. The self-care mental health management apparatus of claim 10, wherein the content delivery unit is configured to analyze the mental health profile of the user based on the user's gender, age, environment, situation, personality, amount of exercise/activity, type of stress management, mental illness history, and mental health-related symptom.

17. The self-care mental health management apparatus of claim 10, wherein the content delivery unit is configured to:
provide a mission for mental health management to the user, and providing a raffle ticket for the user to participate in a raffle for mental health hero characters when the user completes the mission using the user terminal,
provide one or more mental health hero characters of a plurality of predetermined mental health hero characters to the user terminal if the user wins the raffle based on the raffle ticket, and
provide a reward to the user when the user has collected all mental health hero characters included in a preset collection of mental health hero characters.
